# EUROPEAN PATENT SPECIFICATION

(11) **EP 4 286 370 B1**
(45) Date of publication and mention of the grant of the patent: **21.10.2026**
(21) Application number: 21922128.0
(22) Date of filing: 27.05.2021
(51) Int. Cl.: A61K 31/155, A61K 31/40, C07D 207/12, A61P 3/10, A61P 3/00

(54) **USE OF ANISOMYCIN AND DERIVATIVES THEREOF AS GLP-1R AGONISTS**
VERWENDUNG VON ANISOMYCIN UND DERIVATEN DAVON ALS GLP-1R-AGONISTEN
UTILISATION DE L'ANISOMYCINE ET DE SES DÉRIVÉS EN TANT QU'AGONISTES DE GLP-1R

(30) Priority: 26.01.2021 CN 202110103556
(43) Date of publication of application: 06.12.2023
(73) Proprietor: Shanxi Medical University, Taiyuan, Shanxi 030001 (CN)
(72) Inventor: ZHANG, Yi, Taiyuan, Shanxi 030001 (CN); XUE, Huan, Taiyuan, Shanxi 030001 (CN); ZHANG, Min, Taiyuan, Shanxi 030001 (CN); LU, ZhiHong, Taiyuan, Shanxi 030001 (CN); ZHI, Linping, Taiyuan, Shanxi 030001 (CN); WEN, Zhitong, Taiyuan, Shanxi 030001 (CN); CUI, Lijuan, Taiyuan, Shanxi 030001 (CN); BAI, Tao, Taiyuan, Shanxi 030001 (CN); LIU, Yunfeng, Taiyuan, Shanxi 030001 (CN)
(74) Representative: Huang, Liwei
(86) International application number: PCT/CN2021/096473
(87) International publication number: WO 2022/160530

(56) References cited:
- WO-A1-2016/088125
- JP-A- S6 289 659
- US-A- 5 463 078
- US-B2- 9 186 393
- SHI ET AL: "Solid-phase synthesis and anti-infective activity of a combinatorial library based on the natural product anisomycin", BIOORGANIC & MEDICINAL CHEMISTRY LETTERS, ELSEVIER, AMSTERDAM NL, vol. 15, no. 18, 15 September 2005 (2005-09-15), pages 4151 - 4154, XP005021115, ISSN: 0960-894X, DOI: 10.1016/J.BMCL.2005.06.004
- WANG B. ET AL: "Blood pressure-lowering effects of GLP-1 receptor agonists exenatide and liraglutide: a meta-analysis of clinical trials", DIABETES, OBESITY AND METABOLISM, vol. 15, no. 8, 1 August 2013 (2013-08-01), GB, pages 737 - 749, XP055922427, ISSN: 1462-8902, DOI: 10.1111/dom.12085
- DONG YAJIE ET AL: "Efficacy and safety of glucagon-like peptide-1 receptor agonists in non-alcoholic fatty liver disease: A systematic review and meta-analysis", CLINICS AND RESEARCH IN HEPATOLOGY AND GASTROENTEROLOGY, vol. 41, no. 3, 1 June 2017 (2017-06-01), FR, pages 284 - 295, XP093335984, ISSN: 2210-7401, DOI: 10.1016/j.clinre.2016.11.009
- TAI TINA Y ET AL: "Antiepileptic action of c-Jun N-terminal kinase (JNK) inhibition in an animal model of temporal lobe epilepsy", NEUROSCIENCE, NEW YORK, NY, US, vol. 349, 22 February 2017 (2017-02-22), pages 35 - 47, XP029964147, ISSN: 0306-4522, DOI: 10.1016/J.NEUROSCIENCE.2017.02.024
- JI LI; YAN HUA FENG; XIN BAI LI; WEI HAN; HUAN QIU LIU; GUO GUANG SHAO;: "Concise synthesis of ()-anisomycin", CHINESE CHEMICAL LETTERS, ELSEVIER, AMSTERDAM, NL, vol. 23, no. 6, AMSTERDAM, NL , pages 647 - 649, XP028505434, ISSN: 1001-8417, DOI: 10.1016/j.cclet.2012.03.029
- SILVIA KADEN; MIRJAM BROCKMANN; HANS‐ULRICH REISSIG: "Syntheses of Enantiomerically Pure 2‐Substituted Pyrrolidin‐3‐ones via Lithiated Alkoxyallenes – An Auxiliary‐Based Synthesis of both Enantiomers of the Antibiotic Anisomycin", HELVETICA CHIMICA ACTA, VERLAG HELVETICA CHIMICA ACTA., HOBOKEN, USA, vol. 88, no. 7, 20 July 2005 (2005-07-20), Hoboken, USA, pages 1826 - 1838, XP071269068, ISSN: 0018-019X, DOI: 10.1002/hlca.200590143
- EDWARD M. ROSSER, SIMON MORTON, KATE S. ASHTON, PHILIP COHEN, ALISON N. HULME: "SYNTHETIC ANISOMYCIN ANALOGUES ACTIVATING THE JNK/SAPK1 AND P38/SAPK2 PATHWAYS", ORGANIC & BIOMOLECULAR CHEMISTRY, ROYAL SOCIETY OF CHEMISTRY, vol. 02, no. 1, 1 January 2004 (2004-01-01), pages 142 - 149, XP009055070, ISSN: 1477-0520, DOI: 10.1039/b311242j
- SCHWARDT OLIVER, VEITH ULRICH, GASPARD CHRISTIANE, JÄGER VOLKER: "Stereoselective Synthesis and Biological Evaluation of Anisomycin and 2-Substituted Analogues", SYNTHESIS, GEORG THIEME VERLAG, STUTTGART, DE., vol. 1999, no. S1, 1 August 1999 (1999-08-01), STUTTGART, DE. , pages 1473 - 1490, XP055953542, ISSN: 0039-7881, DOI: 10.1055/s-1999-3661
- MASAO TOKUDA ET AL: "New total synthesis of (+)-N-methylanisomycin by anodic cyclization of delta-alkenylamine", TETRAHEDRON, ELSEVIER SIENCE PUBLISHERS, AMSTERDAM, NL, vol. 49, no. 12, 1 January 1993 (1993-01-01), AMSTERDAM, NL , pages 2413 - 2426, XP002154403, ISSN: 0040-4020, DOI: 10.1016/S0040-4020(01)86320-1
- CHANG SHEN, CHEN JIAN-YOU, CHUANG YUNG-JEN, CHEN BOR-SEN: "Systems Approach to Pathogenic Mechanism of Type 2 Diabetes and Drug Discovery Design Based on Deep Learning and Drug Design Specifications", INTERNATIONAL JOURNAL OF MOLECULAR SCIENCES, MOLECULAR DIVERSITY PRESERVATION INTERNATIONAL (MDPI), BASEL, CH, vol. 22, no. 1, 1 January 2021 (2021-01-01), Basel, CH , pages 166 - 33, XP055953546, ISSN: 1422-0067, DOI: 10.3390/ijms22010166
- DATABASE Registry STN; ANONYMOUS : "Butanoic acid, (2R,3S,4S)-4-hydroxy-2-[(4-methoxyphenyl)methyl]-3-pyrrolidinyl ester // Butanoic acid, 3-methyl-, (2R,3S,4S)-4-hydroxy-2-[(4-methoxyphenyl)methyl]-3-pyrrolidinyl ester // Butanoic acid, 4-hydroxy-2-[(4-methoxyphenyl)methyl]-3-pyrrolidinyl ester, hydrochloride, [2R-(2.alpha.,3.alpha.,", XP055953561

## Description

### TECHNICAL FIELD

The invention belongs to the technical field of medicine, and particularly relates to a compound for use in treating a GLP-1R-mediated disease.

### BACKGROUND

Glucagon-like peptide-1 (GLP-1) is secreted by L cells in the gastrointestinal tract and exerts a hypoglycemic effect following the intake of glucose and other nutrients. Studies on the physiological effects of GLP-1 have shown that GLP-1 can not only enhance insulin secretion from β-cells, but also reduce glucagon secretion by acting on α-cells, thereby reducing glycogen output; and GLP-1 acts on the central nervous system and stomach to suppress appetite and slow down gastric emptying, thereby reducing β-cell load, etc. However, since GLP-1 is rapidly degraded by dipeptidyl peptidase 4 (DPP-4) in vivo, its duration of action is limited. Therefore, the current GLP-1-based methods to improve blood sugar control include exogenous drugs that mimic the effect of GLP-1 and prolong the activity of endogenous GLP-1. GLP-1 receptor agonists play the hypoglycemic effect based on this.

So far, traditional drugs used clinically to treat type 2 diabetes (T2DM), such as insulin and sulfonylureas, can effectively reduce hyperglycemia, but after blood sugar is controlled in a normal range, can still continue to exert hypoglycemic effects, which leads to a risk of hypoglycemia in T2DM patients. Studies have reported that GLP-1 receptor agonists stimulate insulin secretion in a glucose concentration-dependent manner through the GLP-1 receptor (GLP-1R), which is the greatest advantage of the GLP-1 receptor agonists over other hypoglycemic drugs, so that the risk of hypoglycemia can be avoided. In addition, the GLP-1 receptor agonists have some effects other than hypoglycemic effect, including reducing weight, cardiovascular benefits, improvement of Alzheimer's disease and fatty liver, etc.

As a hormone secreted from intestinal cells stimulated by eating, incretin can regulate the response of insulin to eating, and its insulin secretion-stimulating effect accounts for about 60% of the total insulin secretion after meals, and plays an important role in blood sugar regulation. GLP-1 and glucose-dependent insulinotropic polypeptide (GIP) are two major incretins. Among them, the secretion of the former is significantly reduced in patients with type 2 diabetes (even those with impaired glucose tolerance), so that GLP-1 has become an important therapeutic target. Studies on the physiological effects of GLP-1 have shown that GLP-1 can not only enhance insulin secretion of β-cells, but also reduce glucagon secretion by acting on α-cells, thereby reducing glycogen output; and GLP-1 acts on the central nervous system and stomach to suppress appetite and slow down Gastric emptying, thereby reducing β-cell load, etc. However, since GLP-1 is rapidly degraded by dipeptidyl peptidase 4 (DPP-4) in vivo, its duration of action is limited. Therefore, the current GLP-1-based methods to improve blood sugar control include exogenous drugs that mimic the effect of GLP-1 and prolong the activity of endogenous GLP-1. GLP-1 receptor agonists play a hypoglycemic effect based on this.

After GLP-1(7-36) amide, which serves as the main active form of GLP-1, enters blood circulation, dipeptidyl peptidase IV (DPP-IV) on the blood and cell membrane will rapidly cleave GLP-1(7-36) amide into inactive GLP-1 (9-36), with the half-life being only 1-2 min, so that GLP-1(7-36) amide is not suitable for long-term treatment of type 2 diabetes (T2DM). Further, scientific research has carried out corresponding transformation and modification on the structure of GLP-1 to increase its half-life, so as to prolong the biological effect in vivo. Diabetes is a chronic disease characterized by hyperglycemia due to insufficient insulin secretion (relative or absolute) or insulin action dysfunction. Type 2 diabetes is a progressive disease. The patient's condition deteriorates over time. It is difficult to control blood sugar well for a long time even with conventional drug treatment. Regardless of metformin, sulfonylureas, or insulin monotherapy, it is difficult to achieve the goal of glycosylated hemoglobin (HbA1c) continuously. To explore the reason, it is related to the progressive decline of β-cell function. GLP-1 can also increase insulin synthesis in vivo, promote β-cell proliferation and differentiation, and inhibit β-cell apoptosis. These characteristics may help to reverse or even prevent diabetes.

GLP-1R agonists known in the prior art for the treatment or prevention of diabetes are generally macromolecular polypeptides, such as lixisenatide, liraglutide, and exenatide, which are difficult to synthesize and prepare and are higher in cost; it is needed to inject them to administrate, which leads to poor patient compliance, and the development of small molecule GLP-1R agonists will improve patient compliance and medication convenience, and reduce drug costs, so that the small molecule GLP-1R agonists have broad clinical market prospects. Therefore, it is of great research significance and commercial value to develop a class of GLP-1R agonists based on small molecular structure.

The chemical structure of anisomycin is as follows:

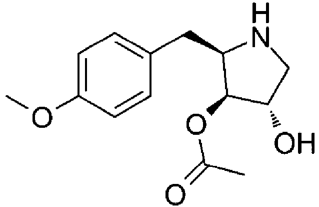

CAS number: 22862-76-6, molecular weight: 265.30, chemical name:
(2R,3S,4S)-2-[(4-methoxyphenyl)methyl]-3,4-pyrrolidinediol 3-acetate methyl ester, which is a pyrrolidine antibiotic separated from Streptomyces griseus. Anisomycin is a protein synthesis inhibitor, which has a good inhibitory effect on some fungi and pathogens, has been successfully used clinically to treat vaginitis caused by trichomonas and dysentery caused by amebic protozoa, etc., and is also widely used to control fungal diseases of plants. Studies have found that anisomycin can significantly inhibit the reactivity of T lymphocytes, with no cytotoxicity found, and can inhibit the rejection reaction of mouse skin transplantation, with its immunosuppressive effect higher than that of the immunosuppressive drug cyclosporin A, and lower toxicity. Therefore, it may be tried for the development of immunosuppressive drugs to treat autoimmune diseases. In addition, anisomycin can activate p38 MAPK and JNKs, and p38 and JNK signaling pathways are involved in various cellular activities, such as cell proliferation and differentiation, apoptosis, etc., suggesting that anisomycin has potential anti-tumor activity and can be used in the development of anti-tumor drugs. The inventor has unexpectedly found that anisomycin has the effect of stimulating GLP-1R, can be used in drugs as the GLP-1R agonist to promote insulin secretion, lower blood sugar, treat diabetes, and has other effects as the GLP-1R agonist. It has not been reported previously that anisomycin and derivatives thereof can stimulate GLP-1R.

US9186393B2 discloses methods and compositions for lowering blood sugar.

WO2016/088125A1 discloses compositions and methods for treating retinal degenerative diseases.

Regarding the effects of GLP-1 receptor agonists, they are reported in the following documents:
Wang B. ET AL: "Blood pressure-lowering effects of GLP-1 receptor agonists exenatide and liraglutide: a meta-analysis of clinical trials", Diabetes, Obesity and Metabolism,vol. 15, no. 8, 1 August 2013 (2013-08-01), pages 737-749.
Dong Yajie ET AL: "Efficacy and safety of glucagon-like peptide-1 receptor agonists in non-alcoholic fatty liver disease: A systematic review and metaanalysis", Clinics and Research in Hepatology and Gastroenterology, vol. 41, no. 3, 1.

### SUMMARY

In order to overcome the disadvantages of synthesis and application of GLP-1R receptor agonists in the prior art because they are all macromolecular polypeptides, the present invention provides use of anisomycin in preparation of a GLP-1R agonist.

The invention is set out in the appended set of claims. The invention provides anisomycin, a compound 1 or a compound 2 as set out in the claims, for use in the treatment of GLP-1R-mediated diseases wherein the GLP-1R-mediated disease is selected from systolic hypertension, cerebral ischemic disease, fatty liver, and dyslipidemia.

The present disclosure provides an anisomycin derivative represented by general formula (I) or pharmaceutically acceptable salts, solvates or stereoisomers thereof, 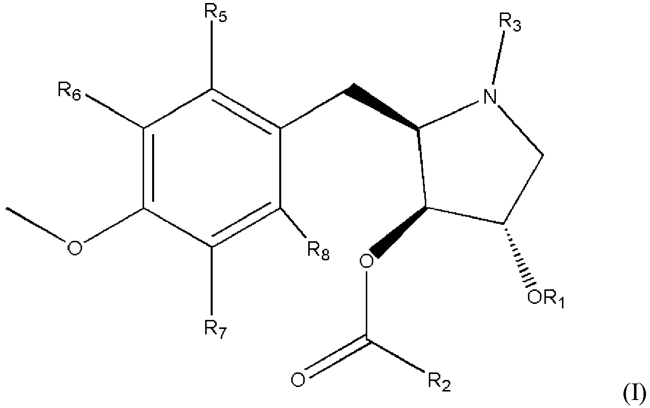 where R₁ is selected from hydrogen, substituted or unsubstituted C1-6 alkyl, and -C(O)R₄; R₂ is selected from hydrogen, substituted or unsubstituted C1-6 alkyl, substituted or unsubstituted C1-6 alkoxy, and -NH-R₄; R₃ is selected from hydrogen, substituted or unsubstituted C1-6 alkyl, substituted or unsubstituted C1-6 alkyl, substituted or unsubstituted C6-12 aryl, substituted or unsubstituted heteroaryl, and -C(O)R₄; R₄ is selected from H, substituted or unsubstituted C1-6 alkyl, and substituted or unsubstituted C1-6 alkoxy; R₅, R₆, R₇, and R₈ are independently selected from halogen atoms, nitro, -COOR₉, CONHR₉, substituted or unsubstituted C1-6 alkyl, and substituted or unsubstituted C1-6 alkoxy; and R₉ is selected from H, and substituted or unsubstituted C1-6 alkyl on condition that the compound of general formula (I) is not anisomycin.

Preferably, the anisomycin derivative provided by the present invention is the following compound 1 and compound 2: 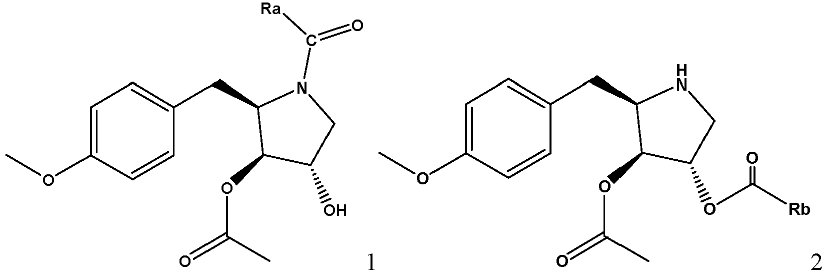 where Ra and Rb are independently selected from substituted or unsubstituted C1-6 alkyl, C1-6 alkoxy, and C6-20 aryl.

The C1-6 alkyl is selected from methyl, ethyl, propyl, and butyl; the C6-20 aryl is selected from phenyl, naphthyl, and anthracenyl; the optional substitution means that any H atom is substituted by hydroxyl, a halogen atom, nitro, C1-4 alkyl, and C1-4 alkoxy.

A GLP-1R agonist, including at least one selected from anisomycin, anisomycin derivatives represented by general formula (I) or pharmaceutically acceptable salts, solvates, or stereoisomers thereof as active ingredients.

The derivative includes products obtained from some group substitutions or reactions by taking anisomycin as the parent, such as substances substituted by halogen atoms, alkyl, alkenyl, alkynyl, alkoxy, hydroxyl, aryl or heteroaryl, or products obtained by reacting with other substances with reactive functional groups, such as products of esterification, etherification, amidation, transesterification, etc.; or salts thereof, such as quaternary ammonium salts; or deuterated derivatives thereof, where deuterated hydrogen is not particularly specified, and is preferably active hydrogen, such as hydrogen on amino or hydrogen on hydroxyl.

Use of at least one selected from anisomycin, anisomycin derivatives represented by general formula (I) or pharmaceutically acceptable salts, solvates, or stereoisomers thereof in preparation of the GLP-1R agonist .

Further, the present disclosure provides use of at least one selected from anisomycin, anisomycin derivatives represented by general formula (I) or pharmaceutically acceptable salts, solvates, or stereoisomers thereof in preparation of drugs to treat GLP-1R-mediated diseases.

The GLP-1R-mediated diseases include, but not limited to, obesity, diabetes, systolic hypertension, cerebral ischemic disease, Alzheimer's disease, fatty liver, dyslipidemia, and neuroprotection.

Preferably, the diabetes is selected from type 2 diabetes.

The inventor has unexpectedly found that anisomycin and the derivative thereof have the function of GLP-1R agonists, and can be used as drugs for treating diabetes. Insulin secretion is stimulated in a glucose concentration-dependent manner through the GLP-1 receptor (GLP-1R), that is, the anisomycin and the derivative thereof will only play a role in promoting insulin secretion at a certain concentration of blood sugar, and will not play a role at a lower blood sugar concentration. Therefore, while treating diabetes, hypoglycemia can also be avoided, which is the benefit of the GLP-1R agonist as a drug for treating diabetes compared with conventional drugs.

Further, the present disclosure provides the GLP-1R agonist taking at least one selected from anisomycin, anisomycin derivatives represented by general formula (I) or pharmaceutically acceptable salts, solvates, or stereoisomers thereof as active ingredients, with the dosage of 0.5-50 mg/kg, and preferably, with the dosage of 5-30 mg/kg.

The present disclosure provides a pharmaceutical composition with GLP-1R agonistic activity, including at least one selected from anisomycin, anisomycin derivatives represented by general formula (I) or pharmaceutically acceptable salts, solvates, or stereoisomers thereof as active ingredients.

Further, the present disclosure provides a pharmaceutical composition for treating or preventing diabetes, including at least one selected from anisomycin, anisomycin derivatives represented by general formula (I) or pharmaceutically acceptable salts, solvates, or stereoisomers thereof as active ingredients.

The pharmaceutical composition can further include metformin, SGLT-2 inhibitors, etc. and excipients.

The pharmaceutical composition is used in dosage forms well known in the art, such as tablets, capsules, and injections.

The GLP-1R agonist effect of the anisomycin and derivatives thereof in the present disclosure is dependent on blood sugar concentration. Therefore, the above method of the present disclosure can not only treat or prevent diabetes, but also effectively reduce the occurrence of hypoglycemia.

### BRIEF DESCRIPTION OF DRAWINGS

FIG. 1 shows the effect of anisomycin on insulin secretion in rats.
FIG. 2 shows the effects of different dosages of anisomycin on insulin secretion in rats.
FIG. 3 shows the effects of anisomycin on insulin secretion in rats under different glucose concentrations.
FIG. 4 shows the effect of anisomycin on insulin secretion in rats in the presence or absence of a GLP-1R blocker Exendin (9-39).
FIG. 5 shows ForteBio Octet molecular interaction to detect the affinity of anisomycin and GLP-1R.
FIG. 6A shows a curve of blood glucose change over time in an oral glucose tolerance test; and FIG. 6 B shows the area under the curve of blood glucose.

### DETAILED DESCRIPTION

### Preparation example

By taking anisomycin as raw material, different anisomycin derivatives are obtained according to the following synthetic routes.

| Compound 1 | Ra |
|---|---|
| 1-1 | -Ph |
| 1-2 | -C₂H₅ |
| 1-3 | -4-Cl-Ph |

| Compound 2 | Rb |
|---|---|
| 2-1 | -Ph |
| 2-2 | -C₂H₅ |
| 2-3 | -C₃H₇ |

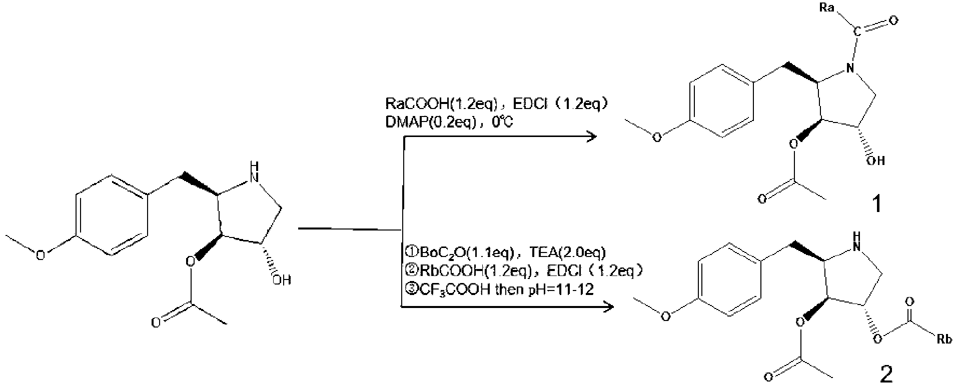

### Example 1

Materials and methods used in the present invention are described below.

### 1.1 Experimental animals

Male Wistar rats, with the body weight of 180-250g, are purchased from the Experimental Animal Center of Shanxi Provincial People's Hospital, where the feeding temperature is 20-22°C, and standard food and drinking water for rodents are equipped. All operating flows meet management and instruction manuals for experimental animals of Shanxi Medical University.

### 1.2 Main drugs and reagents

| Anisomycin | MCE |
|---|---|
| Glucose | Beijing Solarbio Science & Technology Co., Ltd. |
| DispaseII | AMRESCO |
| TEA | Sigma-Aldrich (Shanghai) Trading Co., Ltd. |
| Histopaque-1077 | Sigma-Aldrich (Shanghai) Trading Co., Ltd. |
| Albumin bovine serum (BSA) | Beijing Solarbio Science & Technology Co., Ltd. |
| RPMI 1640 culture medium | HYCLONE |
| Collagenase P | Roche Pharma(Schweiz) Ltd. |
| Fura 2-AM | DOJINDO |
| Fetal calf serum (FBS) | Thermo Fisher |
| Hanks buffer solution (HBSS) | BOSTER Biological Technology Co. Ltd. |
| Analytically pure NaCl | BBI Life Sciences Corporation |
| Analytically pure KCl | BBI Life Sciences Corporation |
| NaHCO₃ | BBI Life Sciences Corporation |
| HEPES | Beijing Solarbio Science & Technology Co., Ltd. |
| CaCl₂ | BBI Life Sciences Corporation |
| Analytically pure MgCl₂ | BBI Life Sciences Corporation |
| KH₂PO₄ | BBI Life Sciences Corporation |
| 100* mycillin mixed solution | Beijing Solarbio Science & Technology Co., Ltd. |
| DMSO | Beijing Solarbio Science & Technology Co., Ltd. |
| MgSO₄ | BBI Life Sciences Corporation |

| 2. Main experimental instruments | |
|---|---|
| Cell incubator | Eppendorf |
| Thermostatic oscillator | Changzhou Guohua Electric Appliance Company Limited |
| Pressure steam sterilizer | Taiyuan Xingyu Scientific and Educational Instrument and Equipment Manufacturing Co., Ltd. |
| Clean bench | Shanghai Zhicheng Analytical Instrument Manufacturing Co., Ltd. |
| Medical refrigerator-freezer | AUCMA |
| Full automatic snowflake ice machine | Changshu Xueke Electric Appliance Company Limited |
| Acidimeter | OHAUS |
| Digital rotating speed peristaltic pump | Baoding Chuangrui Pump Co. Ltd. |
| Inverted microscope | Olympus IX51 |
| VORTEX vortex mixer | Haimen Qilinbeier Instrument Co. Ltd. |

3. Isolation and culture of tissues of pancreas islets of the rats are conventional operations for those skilled in the art.

### 4. Data processing and statistics

Data involved are processed with SigmaPlot12.5 software and are represented by Mean±SEM. Statistic analysis is performed with Student's t-test, one-way ANOVA or paired t-test. In a case where p<0.05, it is of statistical significance.

### Specific operations

### 5.1 Influence of anisomycin on secretion of rat insulin (Reference Example)

Preparation before experiments: 1) there were four groups of experiments, and Ep (Eppendorf) tubes were numbered (7 for each group); 2) a KRBH solution was prepared and was placed in an incubator for inculcation for 30 min, and the pH value was regulated with NaOH to 7.4; and 3) a sample to be tested was prepared from a 2.8mmol/L glucose solution (2.8G), 2.8G+10µM anisomycin, a 11.1 mmol/L glucose solution (11.1G) and 11.1G+10µM anisomycin.

Experimental steps: 1) 500 µL of 2.8G was added into each Ep tube, 5 pancreas islets (uniform in size and smooth in edge) were picked under a stereomicroscope and were placed in the Ep tube, and the Ep tube was placed in the incubator for incubation for 30 min; 2) a supernate was sucked out and abandoned with a pipette, where the pancreas islets were not sucked out, and then 500 µL of 2.8G, 2.8G+10µM anisomycin, 11.1G and 11.1G+10µM anisomycin were added into each group in sequence, and the Ep tube was placed in the incubator for incubation for 30 min; and 3) the supernate was sucked out with the pipette to the Ep tube marked in advance, the mixture was uniformly mixed, and the Ep tube was sealed and preserved at 4°C. The content of insulin in each group is detected by insulin radioimmunoassay.

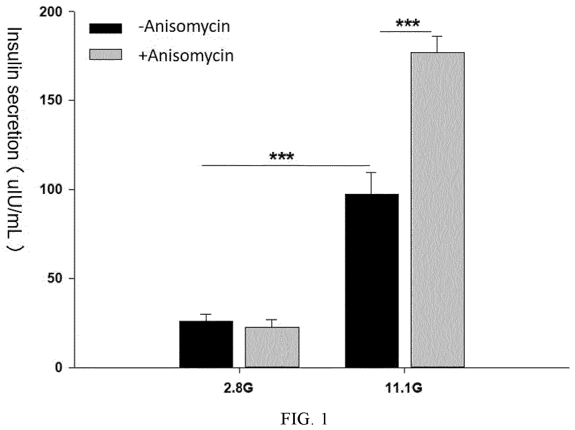

The experimental results are shown in FIG. 1, with the influence of anisomycin on secretion of rat insulin, where +anisomycin represents addition of a certain amount of anisomycin, and +anisomycin in the following figures represents the same meaning. The pancreas islets of the rats are respectively incubated at low glucose concentration (2.8G) and high glucose concentration (11.1G) with anisomycin (10µM). n=7. ***P<0.001. At the basic glucose concentration (2.8G), anisomycin does not have the effect of promoting insulin secretion; at the high glucose concentration (11.1G), anisomycin has the significant effect of promoting insulin secretion. It indicates that anisomycin promotes insulin secretion at high glucose concentration.

### 5.2 Influence of anisomycin with different dosages on secretion of rat insulin (Reference Example)

Preparation before experiments: 1) there were four groups of experiments, and Ep (Eppendorf) tubes were numbered (7 for each group); and 2) a sample to be tested was prepared from a 2.8 mmol/L glucose solution (2.8G), a 11.1 mmol/L glucose solution (11.1G), 11.1G+0.1µM anisomycin, 11.1G+1µM anisomycin, and 11.1G+10µM anisomycin.

Experimental steps: 1) pre-incubation was performed at 2.8G for 30 min; 2) the Ep tube was taken out, the supernate was abandoned, and then 500 µL of 11.1G, 11.1G+0.1µM anisomycin, 11.1G+1µM anisomycin and 11.1G+10µM anisomycin were added into each group in sequence, and the Ep tube was placed in the incubator for incubation for 30 min; and 3) the supernate was sucked out with the pipette to the Ep tube marked in advance, the mixture was uniformly mixed, and the Ep tube was sealed and preserved at 4°C. The content of insulin in each group is detected by insulin radioimmunoassay.

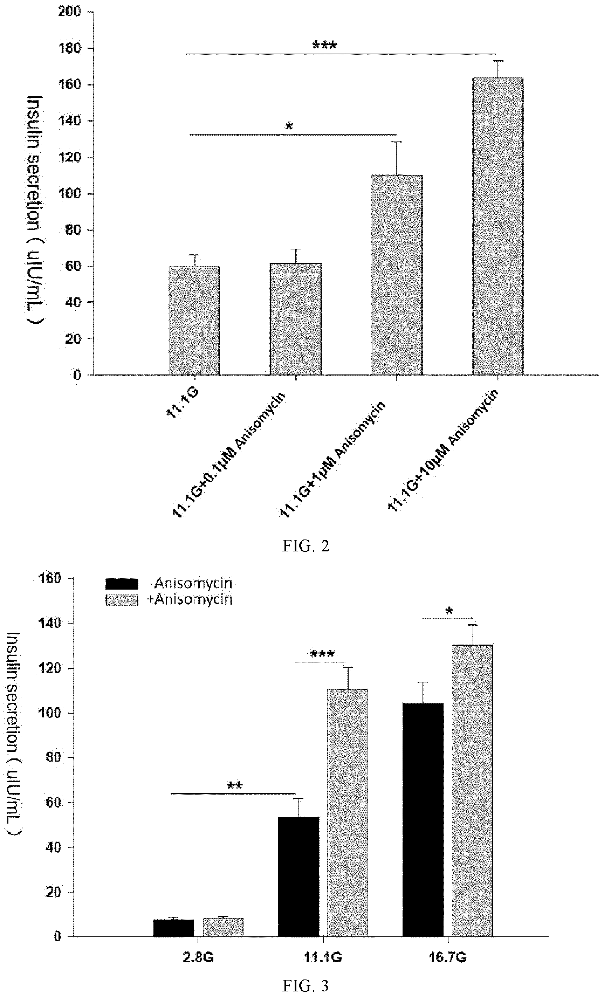

The experimental results are shown in FIG. 2, with influence of anisomycin with different dosages on secretion of rat insulin. At the 11.1 mmol/L glucose concentration (11.1G), anisomycin with different concentrations (0.1µM, 1µM and 10µM) are used to incubator the pancreas islets of the rats. n=7. *P<0.05; ***P<0.001.Compared with independent administration of 11.1 mmol/L glucose (11.1G), with increase of the concentration of anisomycin, the effect of anisomycin promoting insulin secretion is gradually enhanced, and when the concentration of anisomycin is 1 µM and 10 µM, anisomycin has the significant effect of promoting insulin secretion. Therefore, anisomycin has the concentration-dependent effect of promoting insulin secretion at the high glucose concentration (11.1G).

### 5.3 Influence of anisomycin at different glucose concentrations on insulin secretion of the pancreas islets of the rats (Reference Example)

Preparation before experiments: 1) there were three groups of experiments, and Ep tubes were numbered (7 for each group); and 2) a sample to be tested was prepared from a 2.8 mmol/L glucose solution (2.8G), 2.8G+10µM anisomycin, a 11.1 mmol/L glucose solution (11.1G), 11.1G+10µM anisomycin, a 16.7 mmol/L glucose solution (16.7G) and 16.7G+10 µM anisomycin.

Experimental steps: 1) pre-incubation was performed at 2.8G for 30 min; 2) the Ep tube was taken out, the supernate was abandoned, and then 500 µL of 2.8G, 11.1G and 16.7G were added into each group in sequence, and the Ep tube was placed in the incubator for incubation for 30 min; 3) the supernate was sucked out with the pipette to the Ep tube marked in advance, the mixture was uniformly mixed, and the Ep tube was sealed and preserved at 4°C, and 500 µL of 2.8G+10µM anisomycin, 11.1G+10µM anisomycin and 16.7G+10µM anisomycin were added in sequence according to the dosing sequence in the previous round, and the Ep tube was placed in the incubator for incubation for 30 min; and 4) the Ep tube was taken out, the supernate was sucked out to the numbered Ep tubE, the mixture was mixed, and the Ep tube was sealed and preserved at 4 °C. The content of insulin in each group is detected by insulin (INS) radioimmunoassay.

The experimental results are shown in FIG. 3, with the influence of anisomycin at different glucose concentrations on secretion of rat insulin. At the 2.8 mmol/L glucose concentration (2.8G), the 11.1 mmol/L glucose concentration (11.1G) and the 16.7 mmol/L glucose concentration (16.7G), the pancreas islets of the rats are incubated with 10µm anisomycin. n=7,*p<0.5,**p<0.01,***p<0.001. It can be seen that at the basic glucose concentration (2.8G), anisomycin does not have the effect of promoting secretion of insulin; at the concentration of the 11.1 mmol/L glucose solution (11.1G), anisomycin has the significant effect of promoting secretion of insulin; with increase of glucose concentration, at the concentration of the 16.7mmol/L glucose solution (16.7G), anisomycin can still promote secretion of insulin with significant difference. Therefore, anisomycin has the glucose concentration-dependent effect of promoting insulin secretion. It can be illustrated that since anisomycin has the blood sugar concentration dependence, it still can avoid hypoglycemia when being used to treat or prevent diabetes.

### Example 2 Effect of GLP-1 on regulating secretion of rat insulin by ranisomycin

### 1.1 Test reagents

| Exendin (9-30) | MCE |
|---|---|
| Bg1II, NotI, NsiI, HindIII | Fermentas |
| Tween-20 | Beijing Solarbio Science & Technology Co., Ltd. |
| Medium extraction kit | Hangzhou Axygen Biotechnology Co., Ltd. |
| DNA gel extraction kit | Tiangen Biotechnology (Beijing) Co., Ltd. |
| M5 Prestained Plus Protein Ladder | Mei5 Biotechnology Co.,Ltd |
| DNA ligase | Fermentas |
| Biodlight^{™} ECL Chemiluminescent HRP Substrate (High Sensitivity) | Bioworld |
| BamHI, EcoRI, NheI, DNA marker | Fermentas |
| Smal, XhoL, EcoRV | Millipore |
| DNA polymerase | Sangong Biotech |
| High purity plasmid extraction mini kit | Tiangen Biotechnology (Beijing) Co., Ltd. |
| Tryptone and yeast extract | Theromo Fisher |
| Dialytic E.coli cell-free recombinant protein expression kit | Hangzhou Jinao Biotechnology Co., Ltd. |
| Dialytic TOB cell-free recombinant protein expression kit | Hangzhou Jinao Biotechnology Co., Ltd. |
| Dialytic Yeast cell-free recombinant protein expression kit | Hangzhou Jinao Biotechnology Co., Ltd. |
| ClonExpress,^{®}Entry One Step Cloning Kit | Vazyme Biotech Co.,Ltd |

### 1.2 Main experimental instruments

| | |
|---|---|
| PCR meter | Bio-rad |
| Gel imaging system | Bio-rad |
| Western blot vertical electrophoresis system | Bio-rad |
| Western blot half-dry transmembrane system | Bio-rad |
| Octet RE96E molecular interaction analyzer | Fortebio |
| Balance | Shanghai Tianmei Scientific Instrument Co., Ltd. |
| Pipette | Eppendorf Research plus set |
| SSA chip | Fortebio |
| Acidimeter | OHAUS |
| Digital rotating speed peristaltic pump | Baoding Chuangrui Pump Co. Ltd. |
| VORTEX vortex mixer | Haimen Qilinbeier Instrument Co. Ltd. |

### 2.1 Effect of GLP-1 on regulating insulin secretion by ranisomycin (Reference Example)

Preparation before experiments: 1) there were four groups of experiments, and Ep tubes were numbered (7 for each group); and 2) a sample to be tested was prepared from a 2.8 mmol/L glucose solution (2.8G), a 11.1 mmol/L glucose solution (11.1G), 11.1G+100nM Exendin(9-39), 11.1G+10µM ranisomycin, and 11.1G+100nM Exendin(9-39)+10µM ranisomycin.

Experimental steps: 1) pre-incubation was performed at 2.8G for 30 min; 2) the Ep tube was taken out, the supernate was abandoned, and then 500 µL of 11.1G, 11.1G+100nM Exendin(9-39), 11.1G+10µM anisomycin and 11.1G+100nM Exendin(9-39)+10µM anisomycin were added into each group in sequence, and the Ep tube was placed in the incubator for incubation for 30 min; and 3) the supernate was sucked out with the pipette to the Ep tube marked in advance, the mixture was uniformly mixed, and the Ep tube was sealed and preserved at 4°C. The content of insulin in each group is detected by insulin radioimmunoassay.

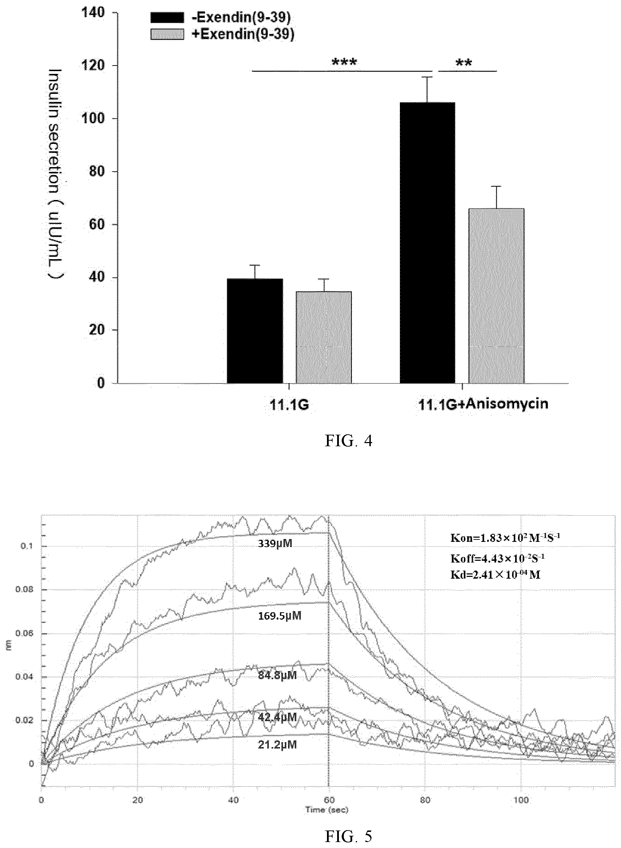

The experimental results are shown in FIG. 4, with the effect of GLP-1 on regulating insulin secretion by ranisomycin. At the 11.1 mmol/L glucose concentration (11.1G), 10µm anisomycin and 100nM Exendin(9-39) are used to incubator the pancreas islets of the rats. n=7,**p<0.01,***p<0.001. Exendin(9-39) is a specifically competitive GLP-1R agonist, which can block the agonistic action of the GLP-1R agonist to GLP-1R. At the concentration of the 11.1 mmol/L glucose solution (11.1G), Exendin(9-39) blocks the effect of anisomycin promoting insulin secretion, indicating that the effect of anisomycin promoting insulin secretion is related to the agonistic GLP-1R.

### 2.2 ForteBio Octet molecular interaction to detect the affinity of anisomycin and GLP-1R

Expression of a cell-free GLP-1R protein: 1) construction of a rat GLP-1R vector: a sequence fragment of rat GLP-1R was obtained by a PCR amplification technique, the GLP-1R rat gene fragment was recovered with an agarose gel, a target gene GLP-1R was cloned to a vector pEX-3, pEX-3 was di-cleaved with EcoRI and BamHI, and was subjected to electrophoresis to recover the vector pEX-3; the amplified fragment are recombined and cloned to the pEX-3 vector by means of ClonExpress^{®} Entry One Step Cloning Kit; the recombinant joint product was converted into a competent cell, clonal colonies were picked and a small amount of the clonal colonies were extracted to obtain plasmids, the plasmids were subjected to di-cleaved identification with EcoRI and BamHI, the plasmids were subjected to electrophoresis, and positive clones were picked; a large amount of recombinant plasmids subjected to sequencing verification were extracted, a bacteria solution corresponding to the positive clones was sequenced, and the residual bacteria solution was preserved with glycerinum; the sequencing result was compared with the target gene sequence, after corrected verification, it was inoculated to an LB culture medium with a glycerinum bacteria solution, and a large amount of plasmids were extracted to obtain an enough amount of recombinant plasmids; and 2) expression of the cell-free GLP-1R protein: the target gene was amplified with high fidelity DNA polymerase G-POL-001 or Z2 ultra fidelity DNA polymerase G-POL-002 purchased from Hangzhou Jinao Biotechnology Co. Ltd. To improve the success rate of protein expression, an E.coli dialytic cell-free protein expression system, a TOB dialytic cell-free protein expression system and a Yeast dialytic cell-free protein expression system were used for expression, and finally, the expression result was observed with SDS-PAGE.

GLP-IR protein purification and concentration: 1) a Histrap column was used to purify a target protein; and 2) a purified sample was acquired, and SDS-PACE was performed to observe a purification result.

Experimental steps of ForteBio Octet molecular interaction: 1) biotinylation treatment was performed on the GLP-1R protein, a proper amount of a biotinylation reagent was added for a water bath reaction at 30°C for 2 h, residual biotins were removed by a gravity desalting column after the reaction, and the mixture was eluted with PBS for SSA chip curing; 2) the compound was dissolved with DMSO, was diluted 100 times after being fully dissolved, and was then diluted with 1%DMSO+PBST to 21.2µM, 42.4µM, 84.8µM and 169.5µM, 339µM, respectively for follow-up detection; 3) the above samples and reagents were added into a sample board in sequence; and 4) a program was set for detection.

Kₒₙ is the association rate constant, which reflects the speed of intermolecular binding; K_{off} is the dissociation rate constant, which reflects the speed of intermolecular dissociation; Kd is the dissociation constant, which reflects the amplitude of affinity of the compound to the target, where the less the value is, the higher the affinity is. Kd=K_{off}/Kₒₙ. For small molecules (<2 kDa), the value Kd is 10⁻⁴-10⁻⁷M, which shows higher affinity. The results are shown in FIG. 5. The ForteBio Octet intermolecular interaction result shows that Kd of anisomycin is equal to 2.41 × 10⁻⁰⁴M, which shows that anisomycin has higher affinity with GLP-1R.

It can be seen from the data in the examples 1 and 2 that anisomycin can effectively promote insulin secretion at higher blood glucose concentration, with certain blood glucose concentration dependence. Moreover, under the action of the GLP-1R agonist, the effect of promoting insulin secretion is reduced or disappears, indicating that GLP-1R mediates the effect of anisomycin promoting insulin secretion. According to the present invention, through ForteBio Octet molecular interaction detection, it is verified that anisomycin and GLP-1R have apparent interaction and higher affinity. The above data can fully show that anisomycin is an effective small molecule GLP-1R agonist.

### Example 3 Influence of anisomycin on blood glucose of C57BL/6 mice (Reference Example)

### 1. Materials and methods

### 1.1 Experimental animals

Male C57BL/6 mice, with the body weight of 18-22 g, are purchased from the Experimental Animal Center of Shanxi Provincial People's Hospital, where the feeding temperature is 20-22°C, and standard food and drinking water for rodents are equipped. All operating flows meet management and instruction manuals for experimental animals of Shanxi Medical University.

### 1.2 Main drugs and reagents

| Anisomycin | MCE |
|---|---|
| Glucose | Beijing Solarbio Science & Technology Co., Ltd. |
| DMSO | Beijing Solarbio Science & Technology Co., Ltd. |
| PEG-200 | Beijing Solarbio Science & Technology Co., Ltd. |

### 1.3 Main experimental instruments

| | |
|---|---|
| Animal balance | Shanghai Fixal Tools Co. Ltd. |
| FreeStyle glucometer and blood glucose test strip | Abbott Diabetes Care Ltd. |
| Low-temperature Ultra-high speed centrifugal machine | Eppendorf |
| Full automatic snowflake ice machine | Changshu Xueke Electric Appliance Company Limited |
| Digital rotating speed peristaltic pump | Baoding Chuangrui Pump Co. Ltd. |
| Micro vortex mixer | Shanghai Huxi Analytic Instrument Co., Ltd. |
| Magnetic stirrer | Shanghai Silei Instrument Co., Ltd. |

### 1.4 Data processing and statistics

Data involved are processed with SigmaPlot12.5 and are represented by Mean±SEM. Statistic analysis is performed with Student's t-test, one-way ANOVA or paired t-test. In a case where P<0.05, it is of statistical significance.

### 2 Experimental results

### 2.1 Oral glucose tolerance test (OGTT)

Drug preparation: anisomycin was dissolved in DMSO, then PEG200 was added, and finally, the mixture was diluted with normal saline (DMSO: PEG200: normal saline=1: 4 : 5).

21 SPF level 6-weeks old C57BL/6 mice were fed adaptively for 1 week and were randomly divided into the following three groups: control group: a solvent (DMSO: PEG200: normal saline=1: 4: 5) was intraperitoneally injected; 5mg/kg anisomycin group: a 5mg/kg anisomycin solution was intraperitoneally injected; and 15mg/kg anisomycin group: a 15mg/kg anisomycin solution was intraperitoneally injected, where the administration volume was 0.1 ml/10g, each group had 7 mice, and the mice drank water and ingested food freely. Before experiments, the mice in each group were subjected to fasting rather than water deprivation for 12 h, gastric irrigation of a 40% glucose solution (2 mg/kg) was performed for each group of mice after administration, blood was sampled from caudal veins in 0 min, 15 min, 30 min, 60 min, 90 min and 120 min after gastric irrigation, blood glucose levels were measured with a glucometer, and the areas under the curve (AUC) of blood glucose were calculated.

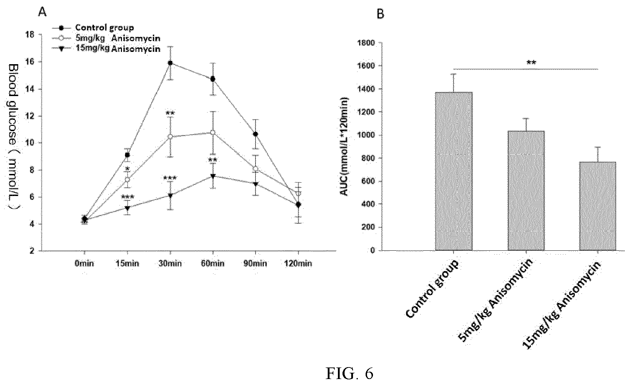

The experimental results are shown in FIG. 6, where A represents a time-varying curve graph of blood glucose in OCTT, and B represents an AUC graph of blood glucose. It can be seen that compared with the control group, after administrating oral glucose, tolerance of the anisomycin administration group to glucose load is improved to different extents, where 15mg/kg anisomycin is more obvious than 5mg/kg anisomycin in glucose reducing effect (n=7, *p<0.05,**p<0.01,***p<0.001).

### Example 4 Influence of anisomycin derivatives on secretion of rat insulin (Reference Example)

Compounds 1-1, 1-2, 2-1 and 2-2 are prepared in the preparation example.

Preparation before experiments: 1) there were ten groups of experiments, and Ep (Eppendorf) tubes were numbered (7 for each group); 2) a KRBH solution was prepared and was placed in an incubator for inculcation for 30 min, and the pH value was regulated with NaOH to 7.4; and 3) a sample to be tested was prepared from a 2.8mmol/L glucose solution (2.8G), 2.8G+10µM compound 1-1, 2.8G+10µM compound 1-2, 2.8G+10µM compound 2-1, 2.8G+10µM compound 2-2, 11.1 mmol/L glucose solution (11.1G), 11.1G+10µM compound 1-1, 11.1G+10µM compound 1-2, 11.1G+10µM compound 2-1 and 11.1G+10µM compound 2-2.

Experimental steps: 1) 500 µL of 2.8G was added into each Ep tube, 5 pancreas islets (uniform in size and smooth in edge) were picked under a stereomicroscope and were placed in the Ep tube, and the Ep tube was placed in the incubator for incubation for 30 min; 2) a supernate was sucked out and abandoned with a pipette, where the pancreas islets were not sucked out, and then 500 µL of 2.8G, 2.8G+10µM compound 1-1, 2.8G+10µM compound 1-2, 2.8G+10µM compound 2-1, 2.8G+10µM compound 2-2, 11.1G, 11.1G+10µM compound 1-1, 11.1G+10µM compound 1-2, 11.1G+10µM compound 2-1 and 11.1G+10µM compound 2-2 were added into each group in sequence, and the Ep tube was placed in the incubator for incubation for 30 min; and 3) the supernate was sucked out with the pipette to the Ep tube marked in advance, the mixture was uniformly mixed, and the Ep tube was sealed and preserved at 4°C. The content of insulin in each group is detected by insulin radioimmunoassay.

The experimental results are shown in table 1, with influence of anisomycin derivatives on secretion of rat insulin. Compared with the 2.8G group, each anisomycin (10µM) does not have the effect of promoting insulin secretion at low glucose concentration (2.8G). Compared with the 11.1G group, each anisomycin (10µM) has the significant effect of promoting insulin secretion at the high glucose concentration (11.1G). (n=7,**p<0.01,***p<0.05)

**Table 1**

| Group | n | Insulin secretion (uIU/mL) |
|---|---|---|
| 2.8G | 7 | 11.0±1.2 |
| 2.8G+compound 1-1 | 7 | 12.6±1.3 |
| 2.8G+compound 1-2 | 7 | 11.9±1.3 |
| 2.8G+compound 2-1 | 7 | 13.0±1.6 |
| 2.8G+compound 2-2 | 7 | 11.6±1.0 |
| 11.1G | 7 | 43.7±6.0 |
| 11.1G+compound 1-1 | 7 | 92.6±8.7** |
| 11.1G+compound 1-2 | 7 | 107.3±12.5** |
| 11.1G+compound 2-1 | 7 | 86.8±9.4* |
| 11.1G+compound 2-2 | 7 | 96.8±7.1** |

It can be known from the data in Table 1 that, in addition to the significant effect of anisomycin on promoting insulin secretion at the high blood sugar concentration, some anisomycin derivatives also have similar physiological activities. Although the present application has specifically implemented a limited number of derivatives, that is, some group substitutions have been carried out, the essence of the present invention lies in the creative discovery that anisomycin and its derivatives with the same parent nucleus have insulin-promoting effects at high blood glucose concentrations, and this effect is exerted through the stimulation of GLP-1R, indicating that this kind of compound is a kind of effective small molecule GLP-1R agonist.

## Claims

1. A compound for use in treating a GLP-1R-mediated disease as a GLP-1R agonist, wherein the GLP-1R-mediated disease is selected from systolic hypertension, cerebral ischemic disease, fatty liver, and dyslipidemia,
wherein the compound is anisomycin, a compound 1, a compound 2, or pharmaceutically acceptable salt thereof; 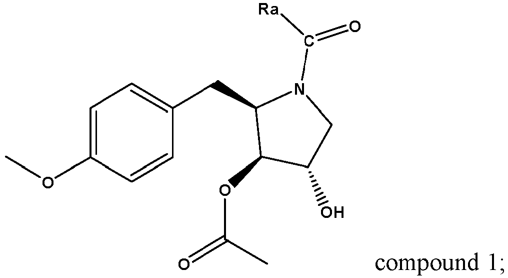 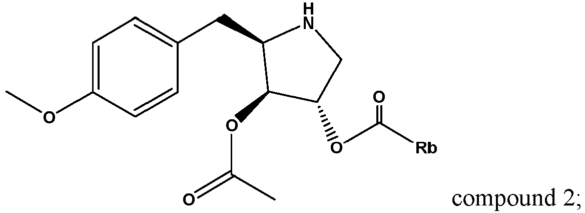
wherein Ra and Rb are independently selected from substituted or unsubstituted C1-C6 alkyl, C1-C6 alkoxyl and C6-C20 aryl.

2. The compound for use according to claim 1, wherein the dosage of the anisomycin, the compound 1, the compound 2, or the pharmaceutically acceptable salt thereof as therapeutic active ingredient is 0.5-50 mg/kg.

3. The compound for use according to claim 2, wherein the dosage of the anisomycin, the compound 1, the compound 2, or the pharmaceutically acceptable salt thereof as therapeutic active ingredient is 5-30 mg/kg.

## Patentansprüche

1. Eine Verbindung zur Verwendung bei der Behandlung einer GLP-1R-vermittelten Erkrankung als GLP-1R-Agonist, wobei die GLP-1R-vermittelte Erkrankung ausgewählt ist aus systolischer Hypertonie, zerebraler ischämischer Erkrankung, Fettleber und Dyslipidämie,
wobei die Verbindung Anisomycin, eine Verbindung 1, eine Verbindung 2 oder ein pharmazeutisch annehmbares Salz davon ist; 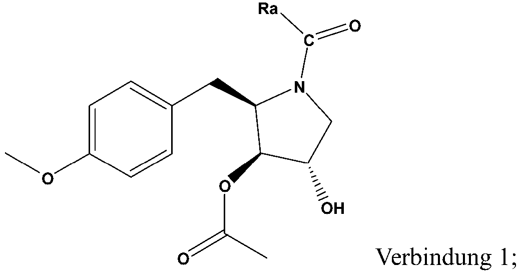 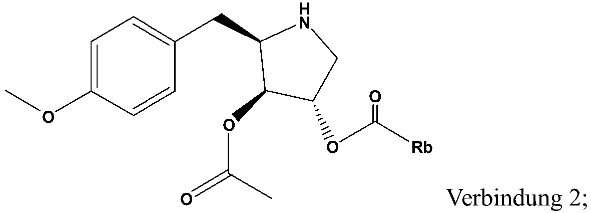
wobei Ra und Rb unabhängig voneinander ausgewählt sind aus substituiertem oder unsubstituiertem C1-C6-Alkyl, C1-C6-Alkoxy und C6-C20-Aryl.

2. Die Verbindung zur Verwendung nach Anspruch 1, wobei die Dosierung des Anisomycins, der Verbindung 1, der Verbindung 2 oder des pharmazeutisch annehmbaren Salzes davon als therapeutisch wirksamer Wirkstoff 0,5 bis 50 mg/kg beträgt.

3. Die Verbindung zur Verwendung nach Anspruch 2, wobei die Dosierung des Anisomycins, der Verbindung 1, der Verbindung 2 oder des pharmazeutisch annehmbaren Salzes davon als therapeutisch wirksamer Wirkstoff 5 bis 30 mg/kg beträgt.

## Revendications

1. Composé pour son utilisation dans le traitement d'une maladie médiée par le récepteur GLP-1 en tant qu'agoniste du récepteur GLP-1, dans lequel la maladie médiée par le récepteur GLP-1 est choisie parmi l'hypertension systolique, la maladie ischémique cérébrale, la stéatose hépatique et la dyslipidémie,
dans lequel le composé est l'anisomycine, un composé 1, un composé 2, ou un sel pharmaceutiquement acceptable de celui-ci ; 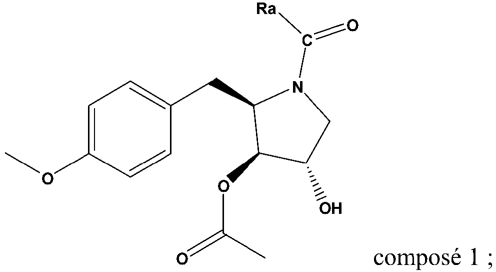 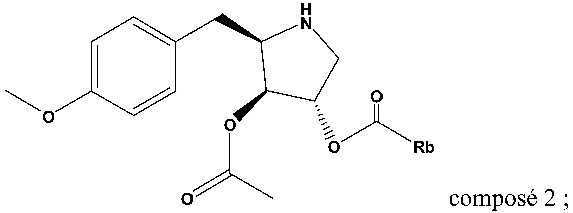
dans lequel Ra et Rb sont indépendamment choisis parmi un alkyle en C1-C6 substitué ou non substitué, un alcoxy en C1-C6 substitué ou non substitué et un aryle en C6-C20 substitué ou non substitué.

2. Composé pour son utilisation selon la revendication 1, dans lequel la dose de l'anisomycine, du composé 1, du composé 2, ou du sel pharmaceutiquement acceptable de celui-ci en tant que principe actif thérapeutique est de 0,5 à 50 mg/kg.

3. Composé pour son utilisation selon la revendication 2, dans lequel la dose de l'anisomycine, du composé 1, du composé 2, ou du sel pharmaceutiquement acceptable de celui-ci en tant que principe actif thérapeutique est de 5 à 30 mg/kg.
